# EUROPEAN PATENT SPECIFICATION

(11) **EP 1 775 601 B1**
(45) Date of publication and mention of the grant of the patent: **16.03.2011**
(21) Application number: 06021475.6
(22) Date of filing: 13.10.2006
(51) Int. Cl.: G01S 15/89, G01S 7/52, A61B 8/00

(54) **System and method for forming 3-dimensional images using multiple sectional plane images**
Vorrichtung und Verfahren zum Bilden von drei-dimensionalen Bilder unter Verwendung von mehreren Schnittbildern
Système et procédé pour former des images tri-dimensionnelles en utilisant de multiples images en coupe

(30) Priority: 17.10.2005 KR 20050097352
(43) Date of publication of application: 18.04.2007
(73) Proprietor: MEDISON CO., LTD., Kangwon-do 250-870 (KR)
(72) Inventor: Kwon, Eui Chul, Gangnam-gu, Seoul 135-280 (KR); Kim, Sung Yun, Gangnam-gu, Seoul 135-280 (KR)
(74) Representative: Schmid, Wolfgang

(56) References cited:
- EP-A2- 0 809 119
- WO-A-00/58754
- WO-A-2005/063125
- WO-A2-02/43801
- US-B1- 6 245 017

## Description

### FIELD OF THE INVENTION

The present invention generally relates to image forming systems, and more particularly to a system and a method for forming a 3-dimensional image by using multiple sectional plane images of a target object.

### BACKGROUND OF THE INVENTION

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation + techniques such as surgery.

The conventional ultrasound diagnostic system acquires 3-dimensional ultrasound data from ultrasound echo signals and forms a 3-dimensional ultrasound image based on the acquired 3-dimensional ultrasound data. In such a case, the entire 3-dimensional ultrasound data are used to form one 3-dimensional ultrasound image, even if the desired portions are different from each other, according to the users' or specific diagnostic purposes. Therefore, such ultrasound diagnostic system is highly inconvenient since the user is required to find a desirable portion in the 3-dimensional ultrasound image.

Also, since the conventional ultrasound diagnostic system uses the entire 3-dimensional ultrasound data to form the 3-dimensional ultrasound image, it takes a very long time to form the 3-dimensional ultrasound image.

US 6,245,017 B1 discloses a three-dimensional ultrasonic diagnostic apparatus which includes a two-dimensional array type of ultrasonic probe. A three-dimensional scan operation of scanning a three-dimensional region within a human body under examination with ultrasound and a two-dimensional scan operation of scanning a two-dimensional plane within the three-dimensional region with ultrasound are selectively performed by a beam former unit. Under the control of a controller, the three-dimensional scan operation is repeated intermittently and the two-dimensional scan operation is repeated during the interval between each three-dimensional scan.

In WO 02/43801 A2 there is described a breast cancer screening system having an ultrasound probe that scans a breast that is flattened along an ultrasound-absorbing plate. The ultrasound probe takes ultrasound image slices from successive planes in the breast volume substantially parallel to a plane of a predetermined x-ray mammogram view of the breast.

An image processing apparatus which can display a tomographic image of an object in a three-dimensional region and a stereoscopic image such as a stereoscopic surface image of the object or a stereoscopic transparent image thereof simultaneously on a screen is known from EP 0 809 119 A2.

WO 2005/063125 A1 relates to a medical system comprising a medical instrument to be guided in a patient body, means for acquiring a 2D X-ray image of said medical instrument, means for acquiring a 3D ultrasound data set of said medical instrument using an ultrasound probe, means for localizing said ultrasound probe in a referential of said X-ray acquisition means, means for selecting a region of interest around said medical instrument within the 3D ultrasound data set, means for converting said first localization into a second localization in said referential of the X-ray acquisition means and means for generating a bimodal representation of said medical instrument detection by combining said 2D X-ray image and the 3D ultrasound data included in said region of interest.

WO 00/58754 A describes a three-dimensional projection image representing a projection of a data volume at a predetermined orientation, three cut plane images representing respective mutually orthogonal planar cuts through the data volume, a graphical representation of the data volume at that orientation and graphical representations of the cut planes are displayed in spaced relationship.

### SUMMARY OF THE INVENTION

The present invention provides a system and a method for setting a region of interest on multiple sectional plane images, extracting data included in the region of interest from 3-dimensional ultrasound image data and performing image rendering for the extracted data, thereby forming a desirable 3-dimensional ultrasound image.

According to one aspect of the present invention, there is provided a system for forming 3-dimensional images according to claim 1.

According to another aspect of the present invention, there is provided a method of forming 3-dimensional images according to claim 3.

### BRIEF DESCRIPTION OF THE DRAWINGS

The above and other objects and features of the present invention will become apparent from the following descriptions of preferred embodiments given in conjunction with the accompanying drawings, in which:
Fig. 1 is a block diagram showing an ultrasound diagnostic system constructed in accordance with the present invention;
Fig. 2 is a flowchart illustrating the operation of an image processor constructed in accordance with the present invention;
Fig. 3 is a schematic diagram showing examples of sectional plane images in 3-dimensional ultrasound image data;
Fig. 4 shows multiple sectional plane images and a reference sectional plane image in accordance with the present invention;
Fig. 5 shows an example of setting a 3-dimensional region on the reference sectional plane image in accordance with the present invention;
Fig. 6 shows an example of slicing a 3-dimensional region on the reference sectional plane image in accordance with the present invention;
Fig. 7 shows an example of displaying slab images in accordance with the present invention; and
Fig. 8 shows an example of displaying the extracted sectional plane images included in the 3-dimensional region selected on a reference sectional image in accordance with the present invention.

### DETAILED DESCRIPTION OF THE PRESENT INVENTION

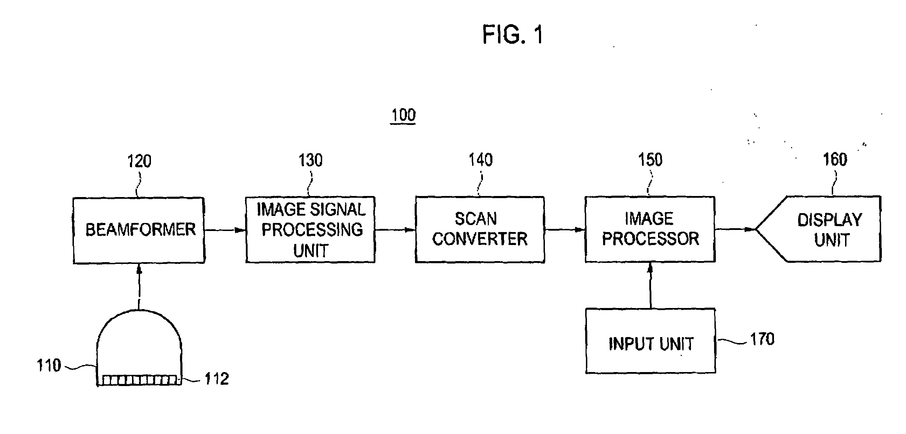

Fig. 1 is a block diagram showing an ultrasound diagnostic system 100, which is constructed in accordance with the present invention. The ultrasound diagnostic system 100 of the present invention includes a probe I 10, a beam former 120, an image signal processor 130, a scan converter 140, an image processor 150, a display unit 160 and an input unit 170. The ultrasound diagnostic system 100 further includes a memory (not shown) for storing 2-dimensional ultrasound image data and 3-dimensional ultrasound image data. Also, the image signal processor 130 and the image processor 150 may be provided as one processor.

The probe 110 includes a 1-dimensional or a 2-dimensional array transducer 112. The transmit signals, which are appropriately delayed in the beam former 120 to form an ultrasound beam, are transmitted to the array transducer 112. Then, the focused ultrasound beam, which is produced in response to the transmit signals, is transmitted along a scan line of a target object (not shown). The probe 110 receives ultrasound echo signals reflected from the target object and converts the ultrasound echo signals into electrical signals (hereinafter referred to as receive signals). The receive signals are transmitted to the beam former 120.

The beam former 120 provides delays of transmit signals to be transmitted to the array transducer 112 included in the probe 110 such that the ultrasound signals outputted from the array transducer 112 arc focused on a focal point. Further, the beam former 120 focuses the receive signals, which are received from the array transducer 112 included in the probe 110, in consideration of the delays with which the echo signals are arrived at each transducer. It then outputs a focused receive beam representing an energy level of the ultrasound echo signal reflected from the focal point.

The image signal processor 130 (e.g., a digital signal processor (DSP)) performs envelop detection for detecting intensities of the focused receive signals to form ultrasound image data. That is, the image signal processor 130 forms 3-dimensional ultrasound image data based on the receive focused signals acquired from each focal point and position information of a plurality of focal points on each scan line. The 3-dimensional ultrasound image data incl ude coordinates information of each focal point, angle information of each scan line and intensity information of the echo signals received at each focal point.

The scan converter 140 scan-converts the 3-dimensional ultrasound image data to a data format capable of being displayed on a screen of the display unit 160. The image processor 150 forms multiple sectional plane images in response to a plane selecting instruction, which is inputted by a user to determine an image slicing direction of sectional plane images. Also, the image processor 150 forms a partial 3-dimensional ultrasound image corresponding to a predetermined region of the 3-dimensional ultrasound image data in response to a region setting instruction inputted from the user. The 3-dimensional ultrasound image is displayed on the display unit 160.

The input unit 170 receives user's selections to transmit the plane selecting instruction and the region setting instruction to the image processor 150. The input unit 170 may be a mouse, a track ball, a keyboard, a touch pad or the like.

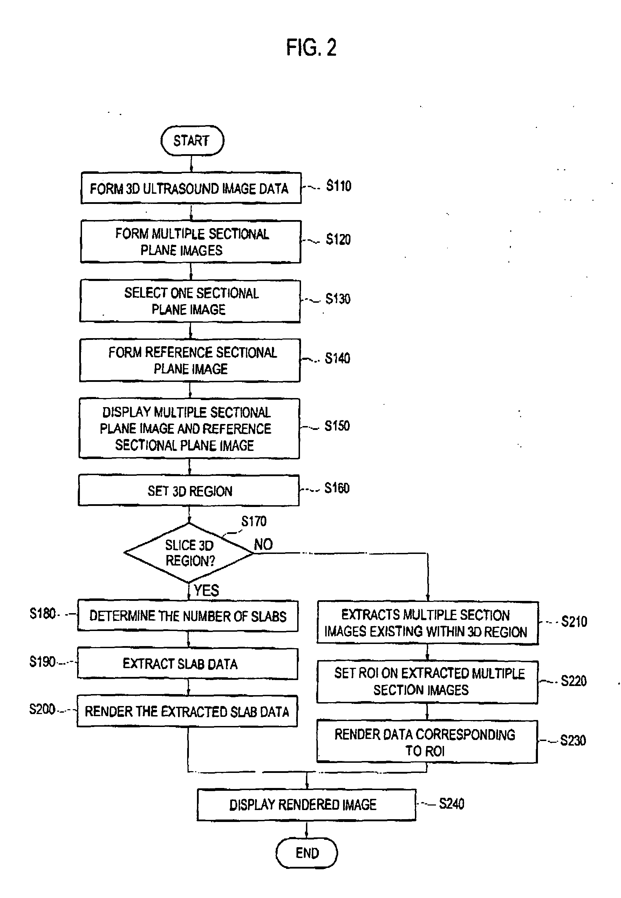

Hereinafter, the operation of the image processor 150 will be described in detail with reference to Figs. 2 to 8. Fig. 2 is a flowchart that shows the operation of the image processor constructed in accordance with the present invention.

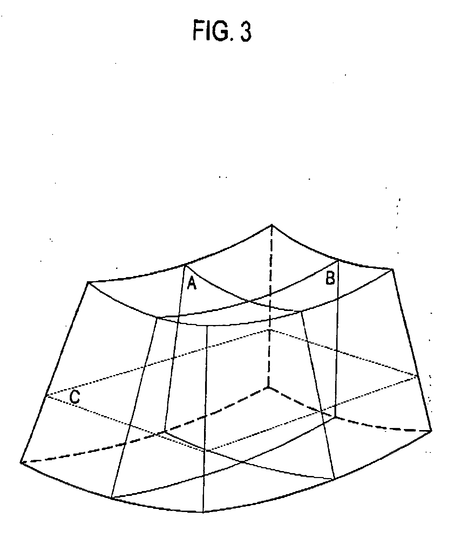

Referring now to Fig. 2, after forming the 3-dimensional ultrasound image data based on the ultrasound echo signals received by the probe 110 at step S 110, the image processor 150 forms multiple sectional plane images in a predetermined image slicing direction, which is determined in response to the plane selecting instruction at step S120. One of the multiple sectional plane images is selected at step S130. The selected sectional plane image may contain images, which are desirable for diagnosis. A reference sectional plane image, which is orthogonal to the selected sectional plane image, is formed at step S140. The reference sectional plane image is used for setting a region representing the width of a partial 3-dimensional ultrasound image (hereinafter referred to as a 3-dimensional region) in the 3-diniensional ultrasound image data. The partial 3-dimensional ultrasound image contains images for diagnosis. For example, as illustrated in Fig. 3, if the selected sectional plane image is an A sectional plane image, then a B sectional plane image or a C sectional plane image becomes a reference sectional plane image. Also, if the selected sectional plane image is the B sectional plane image, then the C sectional plane image or the A sectional plane image becomes a reference sectional plane image. Further, if the selected sectional plane image is the C sectional plane image, then the A sectional plane image or the B sectional plane image becomes a reference sectional plane image.

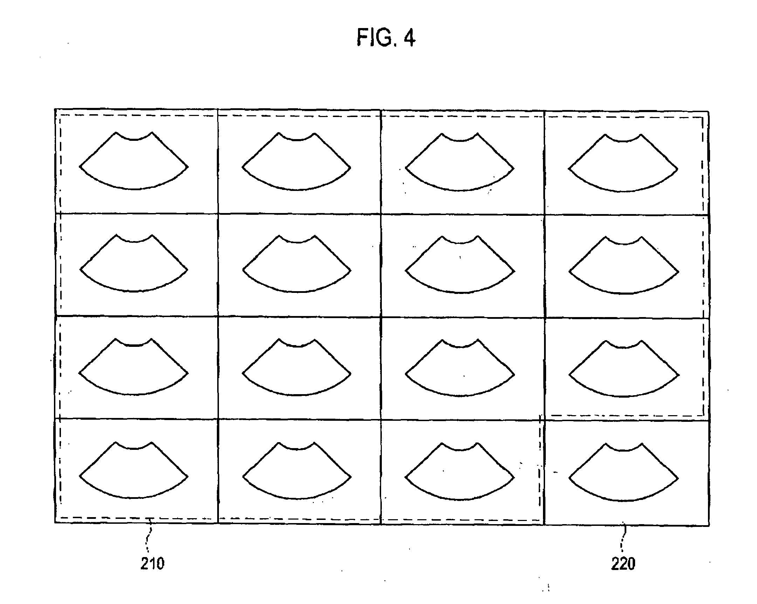

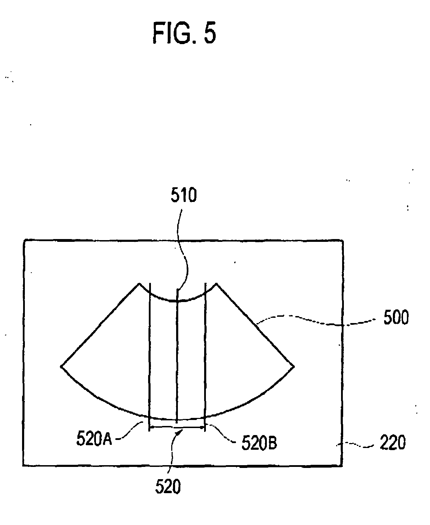

The multiple sectional plane images are displayed on a multiple sectional plane image displaying part 210, whereas the reference sectional plane image is displayed on a reference sectional plane image displaying part 220, as shown in Fig. 4 at step S 150. The 3-dimensional region is set on the reference sectional plane image at step S160. As shown in Fig. 5, in order to set the 3-dimensional region, a center line 510 that represents a position of the selected sectional plane image is indicated on the reference sectional plane image 500. The 3-dimensional region 520 is set with two lines 520A and 520B on the reference sectional plane image 500 to have an identical size at right and left sides of the center line 510. The lines 520 for setting the 3-dimensional region may be straight lines or oblique lines.

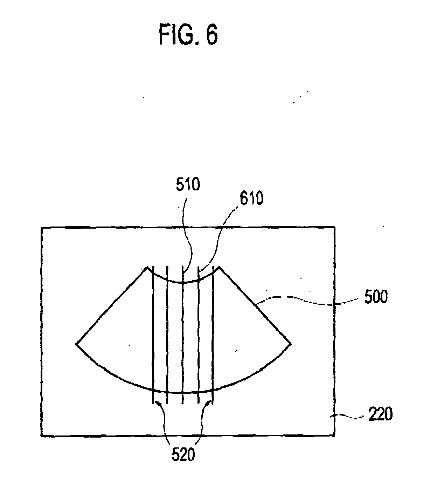

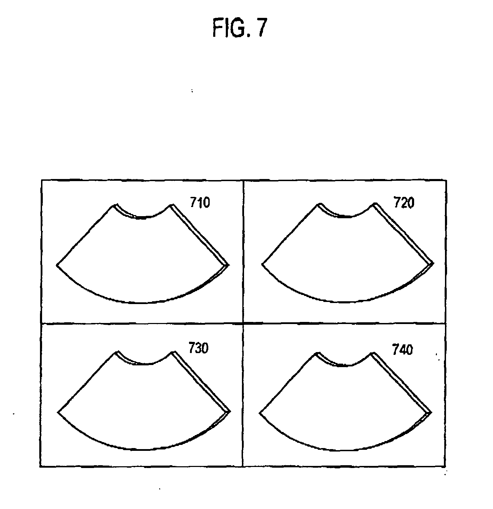

After setting the 3-dimensional region at step S160, it is determined whether to slice the 3-dimensional region at step S170. If it is determined to slice the 3-dimensonal region, then the number of slabs produced by slicing the 3-dimensional region is determined at step S180. Fig. 6 shows the sliced 3-dimensional region 520 on the reference sectional plane image 500. As shown in Fig. 6, four slabs are produced by slicing the 3-dimensional region along slice lines 610 and the center line 510. The width of each slab may be identical to each other in accordance with the preferred embodiment of the present invention. Also, the width of each slab may be adjusted. The image processor 150 extracts slab data corresponding to each of the slabs from the 3-dimensional ultrasound image data at step S 190 and carries out image rendering for the extracted slab data at step S200, thereby forming 3-dimensional slab images 710 to 740 as shown in Fig. 7. The image rendering may be performed by using a volume rendering technique such as a ray casting technique or the like.

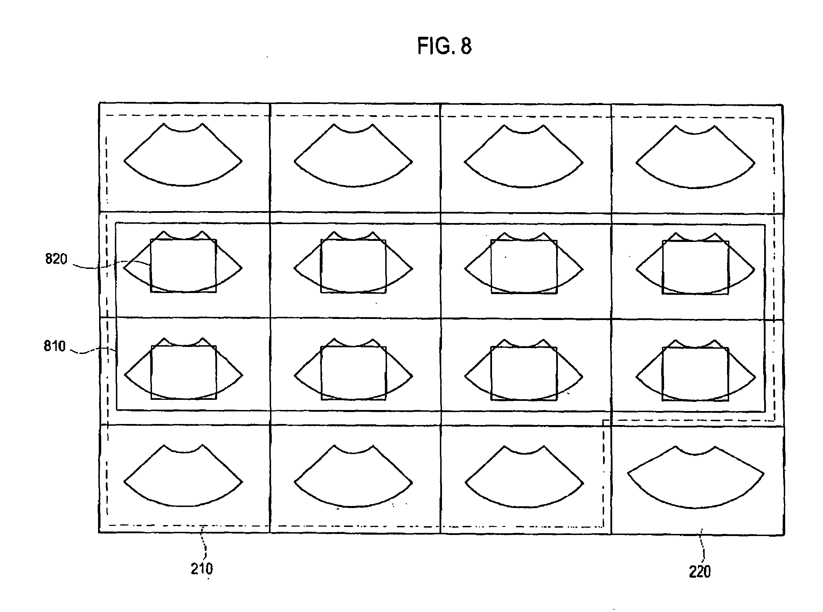

Further, if it is determined not to slice the 3-dimensional region at step S 170, then the image processor 150 extracts multiple sectional plane images included within the 3-dimensional region 520 set by lines 520A and 520B from the multiple sectional plane images shown in Fig. 4. Then, the extracted multiple sectional plane images 810 are selected as shown in Fig. 8 at step S210. Subsequently, a region of interest (ROI) 820 is set on each of the selected multiple sectional plane images 810 at step S220 and then the image processor 150 extracts data corresponding to the ROI from the 3-dimensional ultrasound image data. The image processor 150 carries out an image rendering process for the extracted data at step S230, thereby forming a 3-dimensional ultrasound image.

Since the 3-dimensional region to form the partial 3-dimensional ultrasound image, which contains the images desired for diagnosis, is set on the sectional plane image, the user can easily select the desirable diagnosis region in the 3-dimensional ultrasound image in accordance with the present invention.

Also, since a portion of 3-dimensional ultrasound data is used to form a 3-dimensional ultrasound image, the 3-dimensional ultrasound image can be formed more quickly.

## Claims

1. A system (100) for forming 3-dimensional images, comprising: an image data forming unit (130) for forming 3-dimensional volume data based on image signals, an input unit (170) for receiving a sectional plane selecting instruction and a region setting instruction from a user; an image forming unit (150) for forming multiple sectional plane images sliced along a predetermined direction in a 3-dimensional image, the image forming unit being configured to form at least one 3-dimensional image with partial 3-dimensional volume data selected by using the multiple sectional plane images from the 3-dimensional volume data,
the system (100) being **characterized in that** the image forming unit (150) is adapted to perform:
forming the multiple sectional plane images from the 3-dimensional volume data in the predetermined direction in response to the sectional plane selecting instruction;
forming a reference sectional plane image orthogonal to the multiple sectional plane images from the 3-dimensional volume data;
setting a predetermined region on the reference sectional plane image in response to the region setting instruction;
extracting partial 3-dimensional volume data corresponding to the predetermined region from the 3-dimensional volume data; and
rendering the extracted partial 3-dimensional volume data to form the partial 3-dimensional image; and
displaying the multiple sectional plane images and the 3-dimensional image.

2. The system of Claim 1, wherein the 3-dimensional image is a 3-dimensional ultrasound image.

3. A method of forming 3-dimensional images, comprising the steps of:
a) forming 3-dimensional volume data based on image signals;
b) forming multiple sectional plane images sliced along a predetermined direction from the 3-dimensional volume data,
c) selecting one of the multiple section plane images;
d) forming a reference sectional plane image orthogonal to the selected sectional plane image from the 3-dimensional volume data;
e) setting a predetermined region on the reference sectional plane image;
f) extracting partial 3-dimensional volume data corresponding to the predetermined region from the 3-dimensional volume data;
g) performing image rendering for the extracted partial 3-dimensional volume data; and
h) forming at least one 3-dimensional image.

4. The method of Claim 6, wherein the 3-dimensional image is a 3-dimensional ultrasound image.

5. The method of Claim 6, wherein the step b) includes the steps of:
b1) receiving a sectional plane image selecting instruction to determine a direction of the multiple sectional plane images in the 3-dimensional image; and
b2) forming the multiple sectional plane images in response to the sectional plane image selecting instruction.

6. The method of Claim 8, wherein the step e) includes the steps of:
e1) receiving a region setting instruction to determine a region for forming the 3-diminsional image in the 3-dimensional volume data; and
e2) setting the region by referring to a center line indicating a position of the selected sectional plane image on the reference sectional plane image in response to region setting instruction.

## Patentansprüche

1. System (100) zum Bilden bzw. Erzeugen von 3-dimensionalen Bildern, welches Folgendes aufweist:
eine Bilddatenbildungseinheit (130) zum Bilden von 3-dimensionalen Volumendaten basierend auf Bildsignalen, eine Eingabeeinheit (170) zum Empfangen einer Schnittebenen-Auswahlanweisung und einer Bereichsfestlegeanweisung von einem Benutzer; eine Bild-Bildeeinheit (150) zum Bilden mehrerer Schnittebenenbilder, welche entlang einer vorbestimmten Richtung in einem 3-dimensionalen Bild geschnitten sind, wobei die Bild-Bildeeinheit dafür vorgesehen ist, wenigstens ein 3-dimensionales Bild mit teilweisen 3-dimensionalen Volumendaten zu bilden, welche unter Verwendung der mehreren Schnittebenenbilder von den 3-dimensionalen Volumendaten ausgewählt werden,
wobei das System (100) **dadurch gekennzeichnet ist, dass** die Bild-Bildeeinheit (150) dafür vorgesehen ist, folgendes auszuführen:
Bilden der mehreren Schnittebenenbilder aus den 3-dimensionalen Volumendaten in der vorbestimmten Richtung als Reaktion auf die Schnittebenen-Auswahlanweisung;
Bilden eines Referenzschnittebenenbilds senkrecht zu den mehreren Schnittebenenbilder aus den 3-dimensionalen Volumendaten;
Festlegen eines vorbestimmten Bereichs auf dem Referenzschnittebenenbild als Reaktion auf die Bereichsfestlegeanweisung;
Extrahieren 3-dimensionaler Volumendaten, welche dem vorbestimmten Bereich aus den 3-dimensionalen Volumendaten entsprechen; und
Rendern der extrahierten partiellen 3-dimensionalen Volumendaten, um das partielle 3-dimensionale Bild zu bilden; und
Anzeigen der mehreren Schnittebenenbilder und des 3-dimensionalen Bilds.

2. System nach Anspruch 1, wobei das 3-dimensionale Bild ein 3-dimensionales Ultraschallbild ist.

3. Verfahren zum Bilden bzw. Erzeugen von 3-dimensionalen Bildern, welches die folgenden Schritte aufweist:
a) Bilden von 3-dimensionalen Volumendaten basierend auf Bildsignalen;
b) Bilden von mehrfachen Schnittebenenbildern, welche entlang einer vorbestimmten Richtung aus den 3-dimensionalen Volumendaten geschnitten sind;
c) Auswählen von einem der mehreren Schnittebenenbilder;
d) Bilden eines Referenzschnittebenenbilds senkrecht zu dem ausgewählten Schnittebenenbild aus den 3-dimensionalen Volumendaten;
e) Festlegen eines vorbestimmten Bereichs auf dem Referenzschnittebenenbild;
f) Extrahieren von partiellen 3-dimensionalen Volumendaten, welche dem vorbestimmten Bereich von den 3-dimensionalen Volumendaten entsprechen;
g) Durchführen einer Bildwiedergabe für die extrahierten partiellen 3-dimensionalen Volumendaten; und
h) Bilden wenigstens eines 3-dimensionalen Bilds.

4. Verfahren nach Anspruch 3, wobei das 3-dimensionale Bild ein 3-dimensionales Ultraschallbild ist.

5. Verfahren nach Anspruch 3, wobei der Schritt b) die folgenden Schritte aufweist:
b1) Empfangen einer Schnittebenenbild-Auswahlanweisung, um eine Richtung der mehreren Schnittebenenbilder in dem 3-dimensionalen Bild zu ermitteln; und
b2) Bilden der mehreren Schnittebenenbilder als Reaktion auf die Schnittebenenbild-Auswahlanweisung.

6. Verfahren nach Anspruch 5, wobei der Schritt e) die folgenden Schritte aufweist:
e1) Empfangen einer Bereichsfestlegeanweisung, um einen Bereich zum Bilden des 3-dimensionalen Bilds in den 3-dimensionalen Volumendaten zu ermitteln; und
e2) Festlegen des Bereichs durch Bezugnahme auf eine Mittellinie, welche eine Position des ausgewählten Schnittebenenbilds auf dem Referenzschnittebenenbild als Antwort auf die Bereichsfestlegeanweisung anzeigt.

## Revendications

1. Système (100) pour former des images tridimensionnelles, comprenant:
une unité de formation de données d'images (130) pour former des données de volume tridimensionnel sur la base de signaux d'images, une unité d'entrée (170) pour recevoir une instruction de sélection de plan de coupe et une instruction de réglage de région venant d'un utilisateur, une unité de formation d'images (150) pour former multiples images de plans de coupe effectués le long d'une direction prédéterminée dans une image tridimensionnelle, l'unité de formation d'images étant configurée pour former au moins une image tridimensionnelle avec des données de volume tridimensionnel partiel sélectionnées en utilisant les multiples images de plan de coupe à partir des données de volume tridimensionnel,
le système (100) étant **caractérisé en ce que** l'unité de formation d'images (150) est agencée pour exécuter :
la formation des multiples images de plan de coupe, à partir des données de volume tridimensionnel, dans la direction prédéterminée en réponse à l'instruction de sélection de plan de coupe,
la formation d'une image de plan de coupe de référence orthogonale aux multiples images de plan de coupe, à partir des données de volume tridimensionnel,
le réglage d'une région prédéterminée sur l'image de plan de coupe de référence en réponse à l'instruction de réglage de région,
l'extraction de données de volume tridimensionnel partiel correspondant à la région prédéterminée à partir des données de volume tridimensionnel, et
l'interprétation des données de volume tridimensionnel partiel extraites pour former l'image tridimensionnelle partielle, et
l'affichage des multiples images de plan de coupe et de l'image tridimensionnelle.

2. Système de la revendication 1, dans lequel l'image tridimensionnelle est une image ultrasonore tridimensionnelle.

3. Procédé de formation d'images tridimensionnelles comprenant les étapes de :
a) formation de données de volume tridimensionnel sur la base de signaux d'images,
b) formation de multiples images de plans de coupe effectués le long d'une direction prédéterminée à partir des données de volume tridimensionnel,
c) sélection d'une des multiples images de plans de coupe,
d) formation d'une image de plan de coupe de référence orthogonale à l'image de plan de coupe sélectionnée à partir des données de volume tridimensionnel,
e) réglage d'une région prédéterminée sur l'image de plan de coupe de référence,
f) extraction de données de volume tridimensionnel partiel correspondant à la région prédéterminée à partir des données de volume tridimensionnel,
g) exécution de l'interprétation de l'image pour les données de volume tridimensionnel partiel extraites, et
h) formation d'au moins une image tridimensionnelle.

4. Procédé de la revendication 3, dans lequel l'image tridimensionnelle est une image ultrasonore tridimensionnelle.

5. Procédé de la revendication 3, dans lequel l'étape b) inclut les étapes de :
b1) réception d'une instruction de sélection d'image de plan de coupe pour déterminer une direction des multiples images de plan de coupe dans l'image tridimensionnelle, et
b2) formation des multiples images de plan de coupe en réponse à l'instruction de sélection d'image de plan de coupe.

6. Procédé de la revendication 5, dans lequel l'étape e) inclut les étapes de :
e1) réception d'une instruction de réglage de région pour déterminer une région pour former l'image tridimensionnelle dans les données de volume tridimensionnel, et
e2) réglage de la région en se référant à une ligne médiane indiquant une position de l'image de plan de coup sélectionnée sur l'image de plan de coupe de référence en réponse à l'instruction de réglage de région.
